# EUROPEAN PATENT SPECIFICATION

(11) **EP 1 644 509 B1**
(45) Date of publication and mention of the grant of the patent: **02.05.2007**
(21) Application number: 04741142.6
(22) Date of filing: 19.07.2004
(51) Int. Cl.: C12N 15/864, A61K 48/00, A61P 9/10

(54) **USE OF FAK-RELATED NON-KINASE IN THE MANUFACTURE OF A MEDICAMENT FOR THE INHIBITION OF STENOSIS AND RESTENOSIS**
VERWENDUNG VON FAK VERWANDTEN NICHT KINASE ZUR HERSTELLUNG EINES MEDIKAMENTS ZUR INHIBIERUNG DER STENOSE UND RESTNOSE
UTILISATION DE LA NON-KINASE ASSOCIÉE A LA FAK POUR FABRIQUER UN MÉDICAMENT DESTINÉ A INHIBER UNE STÉNOSE ET UNE RESTÉNOSE

(30) Priority: 17.07.2003 EP 03016259
(43) Date of publication of application: 12.04.2006
(73) Proprietor: Bayerische Julius-Maximilians-Universität Würzburg, 97070 Würzburg (DE)
(72) Inventor: LASER, Martin, 97222 Rimpar (DE); HAUCK, Christof, 97082 Würzburg (DE); STROTMANN, Jörg, 97078 Würzburg (DE)
(74) Representative: Bohmann, Armin K.
(86) International application number: PCT/EP2004/008056
(87) International publication number: WO 2005/014835

(56) References cited:
- WO-A-01/09360
- WO-A-99/28750
- HAUCK CHRISTOF R ET AL: "Focal adhesion kinase facilitates platelet-derived growth factor-BB-stimulated ERK2 activation required for chemotaxis migration of vascular smooth muscle cells" JOURNAL OF BIOLOGICAL CHEMISTRY, vol. 275, no. 52, 29 December 2000 (2000-12-29), pages 41092-41099, XP002303459 ISSN: 0021-9258
- RICHTER M ET AL: "Adeno-associated virus vector transduction of vascular smooth muscle cells in vivo" PHYSIOLOGICAL GENOMICS, AMERICAN PHYSIOLOGICAL SOCIETY, BETHESDA, MD, US, vol. 2, May 2000 (2000-05), pages 117-127, XP002242062 ISSN: 1094-8341
- WALKER HEATHER A ET AL: "Perlecan up-regulation of FRNK suppresses smooth muscle cell proliferation via inhibition of FAK signaling." MOLECULAR BIOLOGY OF THE CELL, vol. 14, no. 5, May 2003 (2003-05), pages 1941-1952, XP002303460 ISSN: 1059-1524
- DATABASE BIOSIS [Online] BIOSCIENCES INFORMATION SERVICE, PHILADELPHIA, PA, US; March 2003 (2003-03), SUNDBERG LIISA J ET AL: "An endogenous inhibitor of focal adhesion kinase blocks Rac1/JNK but not Ras/ERK signaling in vascular smooth muscle cells." XP002303461 Database accession no. PREV200300391683 & FASEB JOURNAL, vol. 17, no. 4-5, March 2003 (2003-03), pages Abstract No. 673.2 URL-http://ww, FASEB MEETING ON EXPERIMENTAL BIOLOGY: TRANSLATING THE GENOME; SAN DIEGO, CA, USA; APRIL 11-15, 2003 ISSN: 0892-6638
- DATABASE BIOSIS [Online] BIOSCIENCES INFORMATION SERVICE, PHILADELPHIA, PA, US; March 2001 (2001-03), TAYLOR JOAN M ET AL: "Selective expression of an endogenous inhibitor of FAK regulates proliferation and migration of vascular smooth muscle cells" XP002303462 Database accession no. PREV200100124318 & MOLECULAR AND CELLULAR BIOLOGY, vol. 21, no. 5, March 2001 (2001-03), pages 1565-1572, ISSN: 0270-7306
- DATABASE MEDLINE [Online] US NATIONAL LIBRARY OF MEDICINE (NLM), BETHESDA, MD, US; 13 October 2000 (2000-10-13), GOVINDARAJAN G ET AL: "Focal adhesion kinase is involved in angiotensin II-mediated protein synthesis in cultured vascular smooth muscle cells." XP002303463 Database accession no. NLM11029408 & CIRCULATION RESEARCH. 13 OCT 2000, vol. 87, no. 8, 13 October 2000 (2000-10-13), pages 710-716, ISSN: 1524-4571
- DATABASE BIOSIS [Online] BIOSCIENCES INFORMATION SERVICE, PHILADELPHIA, PA, US; 1 October 2001 (2001-10-01), HAUCK CHRISTOF R ET AL: "Inhibition of focal adhesion kinase expression or activity disrupts epidermal growth factor-stimulated signaling promoting the migration of invasive human carcinoma cells" XP002303464 Database accession no. PREV200100523969 & CANCER RESEARCH, vol. 61, no. 19, 1 October 2001 (2001-10-01), pages 7079-7090, ISSN: 0008-5472
- SHARIFIL B G ET AL: "Adeno-associated virus-mediated apo A-I milano genetherapy for atherosclerosis and restenosis" JOURNAL OF THE AMERICAN COLLEGE OF CARDIOLOGY, XX, XX, vol. 37, no. 2 Supplement A, February 2001 (2001-02), pages 270A-271A, XP009024833 ISSN: 0735-1097
- MARCH K L ET AL: "THE ADENO-ASSOCIATED VIRUS AS A GENE TRANSFER VECTOR FOR HUMAN AND NON-HUMAN VASCULAR SMOOTH MUSCLE CELLS" EUROPEAN HEART JOURNAL, THE EUROPEAN SOCIETY OF CARDIOLOGY, XX, vol. 13, 1 August 1992 (1992-08-01), page 218, XP002023082 ISSN: 0195-668X

### SEQUENCE LISTING

<110> Bayerische Julius-Maximilians-Universität Würzburg
<120> Use of FAK-related non-kinase for the inhibition of stenosis and restenosis
<130> U 10009 PCT
<160> 4
<170> Patent In version 3.1
<210> 1
   <211> 360
   <212> PRT
   <213> Homo sapiens
<220>
   <221> MISC_FEATURE
   <223> amino acid sequence of human FRNK
<400> 1
   Met Glu Ser Arg Arg Gln Ala Thr Val Ser Trp Asp Ser Gly Gly Ser
   1               5                   10                  15
   Asp Glu Ala Pro Pro Lys Pro Ser Arg Pro Gly Tyr Pro Ser Pro Arg
               20                  25                  30
   Ser Ser Glu Gly Phe Tyr Pro Ser Pro Gln His Met Val Gln Thr Asn
           35                  40                  45
   His Tyr Gln Val Ser Gly Tyr Pro Gly Ser His Gly Ile Thr Ala Met
       50                  55                  60
   Ala Gly Ser Ile Tyr Pro Gly Gln Ala Ser Leu Leu Asp Gln Thr Asp
   65                  70                  75                  80
   Ser Trp Asn His Arg Pro Gln Glu Ile Ala Met Trp Gln Pro Asn Val
                   85                  90                  95
   Glu Asp Ser Thr Val Leu Asp Leu Arg Gly Ile Gly Gln Val Leu Pro
               100                 105                 110
   Thr His Leu Met Glu Glu Arg Leu Ile Arg Gln Gln Gln Glu Met Glu
           115                 120                 125
   Glu Asp Gln Arg Trp Leu Glu Lys Glu Glu Arg Phe Leu Lys Pro Asp
       130                 135                 140
   Val Arg Leu Ser Arg Gly Ser Ile Asp Arg Glu Asp Gly Ser Leu Gln
   145                 150                 155                 160
   Gly Pro Ile Gly Asn Gln His Ile Tyr Gln Pro Val Gly Lys Pro Asp
   165 170 175
   Pro Ala Ala Pro Pro Lys Lys Pro Pro Arg Pro Gly Ala Pro Gly His
   180 185 190
   Leu Gly Ser Leu Ala Ser Leu Ser Ser Pro Ala Asp Ser Tyr Asn Glu
   195 200 205
   Gly Val Lys Leu Gln Pro Gln Glu Ile Ser Pro Pro Pro Thr Ala Asn
   210 215 220
   Leu Asp Arg Ser Asn Asp Lys Val Tyr Glu Asn Val Thr Gly Leu Val
   225 230 235 240
   Lys Ala Val Ile Glu Met Ser Ser Lys Ile Gln Pro Ala Pro Pro Glu
   245 250 255
   Glu Tyr Val Pro Met Val Lys Glu Val Gly Leu Ala Leu Arg Thr Leu
   260 265 270
   Leu Ala Thr Val Asp Glu Thr Ile Pro Leu Leu Pro Ala Ser Thr His
   275 280 285
   Arg Glu Ile Glu Met Ala Gln Lys Leu Leu Asn Ser Asp Leu Gly Glu
   290 295 300
   Leu Ile Asn Lys Met Lys Leu Ala Gln Gln Tyr Val Met Thr Ser Leu
   305 310 315 320
   Gln Gln Glu Tyr Lys Lys Gln Met Leu Thr Ala Ala His Ala Leu Ala
   325 330 335
   Val Asp Ala Lys Asn Leu Leu Asp Val Ile Asp Gln Ala Arg Leu Lys
   340 345 350
   Met Leu Gly Gln Thr Arg Pro His
   355 360
<210> 2
   <211> 360
   <212> PRT
   <213> Mus musculus
<220>
   <221> MISC_FEATURE
   <223> amino acid sequence of murine FRNK
<400> 2
   Met Glu Ser Arg Arg Gln Ala Thr Val Ser Trp Asp Ser Gly Gly Ser
   1               5                   10                  15
   Asp Glu Ala Pro Pro Lys Pro Ser Arg Pro Gly Tyr Pro Ser Pro Arg
               20                  25                  30
   Ser Ser Glu Gly Phe Tyr Pro Ser Pro Gln His Met Val Gln Thr Asn
           35                  40                  45
   His Tyr Gln Val Ser Gly Tyr Pro Gly Ser His Gly Ile Pro Ala Met
       50                  55                  60
   Ala Gly Ser Ile Tyr Gln Gly Gln Ala Ser Leu Leu Asp Gln Thr Glu
   65                  70                  75                  80
   Leu Trp Asn His Arg Pro Gln Glu Met Ser Met Trp Gln Pro Ser Val
                   85                  90                  95
   Glu Asp Ser Ala Ala Leu Asp Leu Arg Gly Met Gly Gln Val Leu Pro
               100                 105                 110
   Pro His Leu Met Glu Glu Arg Leu Ile Arg Gln Gln Gln Glu Met Glu
           115                 120                 125
   Glu Asp Gln Arg Trp Leu Glu Lys Glu Glu Arg Phe Leu Lys Pro Asp
       130                 135                 140
   Val Arg Leu Ser Arg Gly Ser Ile Asp Arg Glu Asp Gly Ser Phe Gln
   145                 150                 155                 160
   Gly Pro Thr Gly Asn Gln His Ile Tyr Gln Pro Val Gly Lys Pro Asp
                   165                 170                 175
   Pro Ala Ala Pro Pro Lys Lys Pro Pro Arg Pro Gly Ala Pro Gly His
               180                 185                 190
   Leu Ser Asn Leu Ser Ser Ile Ser Ser Pro Ala Asp Ser Tyr Asn Glu
           195                 200                 205
   Gly Val Lys Leu Gln Pro Gln Glu Ile Ser Pro Pro Pro Thr Ala Asn
       210                 215                 220
   Leu Asp Arg Ser Asn Asp Lys Val Tyr Glu Asn Val Thr Gly Leu Val
   225 230 235 240
   Lys Ala Val Ile Glu Met Ser Ser Lys Ile Gln Pro Ala Pro Pro Glu
   245 250 255
   Glu Tyr Val Pro Met Val Lys Glu Val Gly Leu Ala Leu Arg Thr Leu
   260 265 270
   Leu Ala Thr Val Asp Glu Thr Ile Pro Ala Leu Pro Ala Ser Thr His
   275 280 285
   Arg Glu Ile Glu Met Ala Gln Lys Leu Leu Asn Ser Asp Leu Gly Glu
   290 295 300
   Leu Ile Ser Lys Met Lys Leu Ala Gln Gln Tyr Val Met Thr Ser Leu
   305 310 315 320
   Gln Gln Glu Tyr Lys Lys Gln Met Leu Thr Ala Ala His Ala Leu Ala
   325 330 335
   Val Asp Ala Lys Asn Leu Leu Asp Val Ile Asp Gln Ala Arg Leu Lys
   340 345 350
   Met Leu Gly Gln Thr Arg Pro His
   355 360
<210> 3
   <211> 1083
   <212> DNA
   <213> Homo sapiens
<220>
   <221> misc_feature
   <223> coding sequence of human FRNK
<400> 3
   atggagtcca gaagacaggc cacagtgtcc tgggactccg gagggtctga tgaagcaccg      60
   cccaagccca gcagaccggg ttatcccagt ccgaggtcca gcgaaggatt ttatcccagc     120
   ccacagcaca tggtacaaac caatcattac caggtttctg gctaccctgg ttcacatgga     180
   atcacagcca tggctggcag catctatcca ggtcaggcat ctcttttgga ccaaacagat     240
   tcatggaatc atagacctca ggagatagca atgtggcagc ccaatgtgga ggactctaca     300
   gtattggacc tgcgagggat tgggcaagtg ttgccaaccc atctgatgga agagcgtcta     360
   atccgacagc aacaggaaat ggaagaagat cagcgctggc tggaaaaaga ggaaagattt     420
   ctgaaacctg atgtgagact ctctcgaggc agtattgaca gggaggatgg aagtcttcag     480
   ggtccgattg gaaaccaaca tatatatcag cctgtgggta aaccagatcc tgcagctcca     540
   ccaaagaaac cgcctcgccc tggagctccc ggtcatctgg gaagccttgc cagcctcagc     600
   agccctgctg acagctacaa cgagggtgtc aagcttcagc cccaggaaat cagcccccct     660
   cctactgcca acctggaccg gtcgaatgat aaggtgtacg agaatgtgac gggcctggtg     720
   aaagctgtca tcgagatgtc cagtaaaatc cagccagccc caccagagga gtatgtccct     780
   atggtgaagg aagtcggctt ggccctgagg acattattgg ccactgtgga tgagaccatt     840
   cccctcctac cagccagcac ccaccgagag attgagatgg cacagaagct attgaactct     900
   gacctgggtg agctcatcaa caagatgaaa ctggcccagc agtatgtcat gaccagcctc     960
   cagcaagagt acaaaaagca aatgctgact gctgctcacg ccctggctgt ggatgccaaa    1020
   aacttactcg atgtcattga ccaagcaaga ctgaaaatgc ttgggcagac gagaccacac    1080
   tga                                                                  1083
<210> 4
   <211> 1083
   <212> DNA
   <213> Mus musculus
<220>
   <221> misc_feature
   <223> coding sequence of murine FRNK
<400> 4
   atggaatcca gaagacaggc taccgtgtcc tgggactctg gagggtctga tgaagcacca     60
   cccaagccca gcagacctgg ttatcctagc ccgagatcca gtgaaggatt ttatcccagc    120
   ccacagcaca tggtccagac caatcactac caggtctctg gctacccggg ttcccatgga    180
   atcccagcca tggctggcag catttaccaa ggtcaggcat ctcttctgga ccagacagaa    240
   ttgtggaatc acagacctca ggagatgtcc atgtggcagc ccagtgtgga ggattcggca    300
   gctttggacc ttcgagggat gggacaggtg cttccccctc acctgatgga agagcggcta    360
   atccgacagc agcaggaaat ggaagaagac cagcgctggc tggagaagga agagcgattc    420
   ctgaaacctg atgtgaggct ctctcgaggc agcatcgaca gggaagacgg gagttttcag    480
   ggtccgactg gaaaccaaca catctatcag cctgtgggga agccagatcc tgcagctcca    540
   ccaaagaaac ctcctcgccc tggagcacct ggccacctaa gcaacctgtc cagcatcagc    600
   agccctgcag acagctacaa tgagggtgtc aagcttcagc cccaggaaat cagcccccct    660
   cccactgcca accttgaccg gtccaatgac aaggtatatg agaatgtgac aggcctagtg    720
   aaggctgtca tcgagatgtc cagcaaaatc cagccagctc ctccagaaga gtacgtccct    780
   atggtgaagg aagttggcct ggccctgcgg accttactag ccacggtgga tgagaccatt    840
   cctgctcttc cagccagcac tcatcgagag atcgagatgg cacagaagct gctgaactcc    900
   gacttaggcg agctcatcag caagatgaag ctggcgcagc agtacgtcat gaccagcctg    960
   cagcaggagt ataagaagca gatgctgaca gctgctcacg ccctggctgt ggatgccaag   1020
   aacctacttg atgttattga tcaagcaaga ctgaaaatgc tagggcagac acggccacac   1080
   tga                                                                 1083

## Description

The present invention is related to compounds for the treatment of coronary and/or peripheral vascular diseases.

Ischaemic heart diseases such as angina pectoris or heart attack are the number one cause of death in the European Union and other industrialized countries. The method of choice for treating this kind of disease is an intervention at the coronary arteries by means of ballooning the arteries and/or implantation of a stent. However, after stent implantation about 20% to 60% of all patients depending on vessel lesions and risk profile, experience a re-occlusion of the vessels due to a restenosis within 6 month of the initial treatment and have thus to undergo revascularisation. The pathophysiological mechanisms underlying restenosis are now basically understood. Typically, restenosis is caused by proliferation and migration of vascular smooth muscle cells (SMC) from the tunica media into the tunica intima which results in the formation of a neointima decreasing the vessel lumen.

In view of this, it is desirable to reduce the risk of restenosis. Approaches of the prior art are based on improvements on the stent design, the use of ultrasound, cold or photochemical substances as well as the use of stents which are covered by radioactive substances. Apart from these physical means, also chemical means are employed to avoid restenosis, whereby particularly preferred are cytostatics as well as anti-inflammatory substances such as Sirolimus, Tacrolimus, Actinomycin D, Taxol, Paclitaxel, Dexamethason, inhibitors to matrix metalloproteases, statins, integrin antagonists, vasodilators and NO-donors.

The study of the use of a particular drug containing stent, namely the sirolimus-eluting Cypher stent (Cordis, Johnson & Johnson), in preventing restenosis, is disclosed in Zeitschrift für Kardiologie, 91, suppl. 3, 2002, 49-57. Possible problems related to thrombosis induction in the use thereof have been noted: see
http://www.fda.gov/bbs/topics/news/cordis_Itr.pdf, and
http://www.fda.gov/bbs/topics/NEWS/2003/NEW00919.html.

A further approach for the treatment of restenosis is based on gene therapy. In the prior art a number of candidate genes such as VEGF, c-myc, p53, eNOS and others, were targeted for that purpose. However, apart from choosing the optimum target, particular problems arose so far from the short contact times between the cytoplasma membranes of the migrating vascular smooth muscle cells and the administered vectors addressing the candidate genes.

The chemical compounds administered for the purpose of treating restenosis are typically applied by and released from so-called drug-eluting stents. However, delivering pharmaceutically active compounds through stents requires that the substances are coated onto the stents, preferably through polymer coatings. This kind of coating results in an increased volume of the stent compared to an uncoated stent and thus an increased risk of inflammation and restenosis. Also, this kind of stent is difficult to manufacture. Still there is a need for another means for the treatment and/or prevention of restenosis and diseases related to or arising from restenosis.

According to a first aspect the problem underlying the present invention is solved by the use of an adeno-associated virus, whereby the adeno-associated virus comprises
a) FRNK or a derivative thereof which retains the functional activity of FRNK, or
b) a nucleic acid encoding FRNK or a derivative thereof which retains the functional activity of FRNK.

In a second aspect the problem underlying the present invention is solved by the use of a nucleic acid coding for an adeno-associated virus, whereby the nucleic acid comprises a nucleic acid encoding FRNK, for the manufacture of a medicament.

In a third aspect the problem underlying the present invention is solved by the use of a nucleic acid coding for an adeno-associated virus, whereby the nucleic acid comprises a nucleic acid encoding FRNK, for the coating of an implant.

In a preferred embodiment of the third aspect of the present invention the use is for the coating of a stent and/or for the coating of a catheter.

In a further embodiment of any of the aspects of the present invention FRNK comprises an amino acid sequence according to SEQ. ID. No. 1 or SEQ. ID. No. 2.

In a still further embodiment of any of the aspects of the present invention FRNK is encoded by a nucleic acid according to SEQ. ID. No. 3 or SEQ. ID. No. 4.

In another embodiment of any of the aspects of the present invention FRNK is encoded by a nucleic acid which hybridises to the nucleic acid sequence according to SEQ. ID. No. 3 or SEQ. ID. No. 4.

In a further embodiment of any of the aspects of the present invention FRNK is encoded by a nucleic acid which, but for the degeneracy of the genetic code, would hybridise to the nucleic acid sequence according to SEQ ID No. 3 or SEQ. ID. No. 4, preferably under stringent conditions.

In a further embodiment of any of the aspects the adeno-associated virus and/or the medicament comprising the adeno-associated virus and/or the adeno-associated virus encoding nucleic acid is administered through a catheter or an implant.

In a preferred embodiment the implant is a stent.

In a fifth aspect the problem underlying the present invention is solved by a stent coated with an adeno-associated virus or a nucleic acid encoding the adeno-associated virus as described in connection with any of the aspects of the present invention.

In a sixth aspect the problem underlying the present invention is solved by a catheter coated with an adeno-associated virus or a nucleic acid encoding the adeno-associated virus as described in connection with any of the aspects of the present invention.

In a seventh aspect the problem underlying the present invention is solved by the use of a stent as described in connection with the fifth aspect of the present invention, in the manufacture of a medicament or in the manufacture of a medical device.

In an eighth aspect the problem underlying the present invention is solved by the use of a catheter as described in connection with the sixth aspect of the present invention, in the manufacture of a medicament or in the manufacture of a medical device.

In a preferred embodiment of the use according to the various aspects of the present invention the medicament and/or the medical device is for the treatment and/or prevention of a coronary and/or peripheral vascular disease.

In an even more preferred embodiment of the use according to the various aspects of the present invention the coronary and/or peripheral vascular disease is selected from the group comprising restenosis, angiogenesis, angina pectoris, heart attack, acute coronary syndrome, peripheral arterial occlusion disease, vasculitis, thrombosis, carotis stenosis, stroke, infarction, gangrene, arterial ulcera, venous thrombosis and amputation of any extremities.

The present inventors have surprisingly found that the combination of using a distinct target which is involved in the restenosis process, and a distinct vector system results in an efficient gene therapy based treatment of restenosis and diseases or conditions related thereto. More particularly, the target is the focal adhesion kinase (FAK), and even more preferred targets are inhibitors thereto. A particularly preferred inhibitor is FRNK. Based on this, the present inventors have perceived to use as distinct vector system adeno-associated viruses which contain or code for an inhibitor of FAK, more particularly those which code for FRNK. Such adeno-associated virus is administered to an organism in need thereof, more particularly to the site where stenosis or restenosis is going to occur or is present, in order to infect the vascular smooth muscle cells involved in this event using the vector system. The particular combination of using adeno-associated virus as delivery vehicle and transfection means for vascular smooth muscle cells and to thus introduce a potent inhibitor to FAK such as FRNK provides for a significant overexpression of the inhibitor and a nearly quantitative gene transfer of the inhibitor coding nucleic acid into the respective cells, preferably into the vascular smooth muscle. The combination of these mechanisms leads to a highly efficient and persistent effect on the pathomechanism related to stenosis and restenosis and the cells involved therein, particularly the vascular smooth muscle cells.

The cellular cause for restenosis is an undesired migration of vascular smooth muscle cells (SMC) from the tunica media into the tunica intima of the respective vessel, more preferably of arteries. Under non-pathological conditions migration of vascular smooth muscle cells is a process which is involved in wound healing processes in the adult organism. Coordinating cellular adhesion to the extracellular matrix (ECM) and the control of contractility of the intracellular actin cytoskeleton are essential for a defined motion of a eukaryotic cell. Integrins are, among others, the cellular receptors for proteins of the extracellular matrix and are accumulated at discrete sites which are referred to as focal adhesions or focal adhesion points. Apart from mediating the interaction of the extracellular matrix and the intracellular actin cytoskeleton, these focal adhesions are essential for signal transduction into the cell, including the control of adhesion dependent growth and regulation of cell motility. As integrins do not have an enzymatic activity themselves, integrin mediated signal transduction relies on the activity of integrin associated enzymes.

One of these integrin associated enzymes is the focal adhesion kinase (FAK) which is a protein tyrosine kinase present in adherent cells such as epithelial cells, fibroblasts or smooth muscle cells. FAK is activated upon cell matrix contact, mechanical stress or addition of different growth factors. Due to an increased enzymatic activity autophosphorylation of the tyrosine residue Y-397 of FAK occurs. Essential to the function of FAK is the C-tenminal located focal adhesion targeting domain (FAT) which mediates the localisation of FAK to integrin rich focal adhesions. In some cell types such as smooth muscle cells the C-terminal region of FAK is expressed as a separate transcript and protein, respectively, which is also referred to as FAK-related non-kinase (FRNK). As FRNK comprises the FAT domain, FRNK is also recruited to the focal adhesions and is competing there with FAK for binding sites. Therefore, FRNK is a dominant-negative version of endogenous FAK and can thus block FAK dependent cellular events. Based on this mechanism, FRNK and its derivatives can be used to inhibit FAK mediated events such as motility of smooth muscle cells and thus to control restenosis and stenosis, respectively.

FRNK and derivatives thereof are defined in preferred embodiments by the amino acid sequence according to SEQ ID No. 1 which is the human FRNK, or the one according to SEQ. ID. No. 2 which is the murine FRNK or the corresponding nucleic acid sequence coding therefore such as SEQ. ID. No. 3 and SEQ. ID. No. 4, respectively. In addition, FRNK as used herein is preferably any FAK derivative which is capable of competing with FAK activity at the focal adhesions without exercising FAK's activity in signal transduction. This kind of activity is also referred to herein as FRNK activity. It is within the skills of the ones of the art to derivatize FAK to such an extent as to provide for this kind of FRNK activity. Therefore, the present invention may use in preferred embodiments any protein having a FRNK activity and any nucleic acid coding therefor. Without wishing to be bound by any theory, however, it seems that any such derivative should comprise the FAT domain of FAK. Preferably, FRNK comprises the amino acid residues 693 to 1050 of the FAK amino acid sequence such as disclosed in databank accession number # Q 05397, i. e. the carboxy terminal 359 amino acids of said FAK. In further embodiments, FRNK also includes fragments of the 359 amino acid carboxy-terminus of FAK that retain at least one binding function or biological function of FRNK, more particularly binding to focal adhesions and comprising the paxillin and talin binding site. Such fragments are preferably 100, 150 or 200 amino acids in length. Such fragments can be formed by deleting an amino terminus segment from FRNK or a protein having FRNK activity, by deleting a carboxy-terminus segment thereof, by deleting an intervening segment thereof, and combinations thereof. It will also be appreciated that one or more minor heterogenous amino acid segments, such as, e. g. 1 to 10 amino acids in length, can be inserted into FRNK or FRNK fragments, at the amino terminus, at the carboxy terminus, in an intervening position, or combinations thereof, without changing the function or activity of FRNK. It will be understood that the FAK and/or FRNK and the respective nucleic acid coding therefor can be those of any species, preferably the species is a mammalian species and even more preferable the mammalian is a human.

A nucleic acid encoding FRNK, as used herein, is preferably the nucleic acid sequence according to SEQ ID No. 3 and/or SEQ. ID. No. 4. In a further embodiment, the FRNK encoding nucleic acid is a nucleic acid which codes for the FRNK protein as described previously, more preferably the FRNK protein according to SEQ ID No. 1 and/or SEQ. ID. No. 2. It is within the skills of the ones of the art to deduce from the amino acid sequence any nucleic acid sequence which, in principle, would encode for said amino acid sequence, particularly given the degeneracy of the genetic code. Particularly preferred nucleic acid sequences are those which make use of the respective codon usage as pertinent to eucaryotic cells, more particularly to smooth muscle cells and even more preferably vascular smooth muscle cells. Also the nucleic acid encoding FRNK and its derivatives is any nucleic acid which hybridizes to the nucleic acid sequence described previously, more preferably the nucleic acid sequences according to SEQ ID No. 3 and/or SEQ. ID. No. 4, or would do so but for the degeneracy of the genetic code. For preferred embodiments of the present invention it is essential that any of the nucleic acids described herein encode for FRNK or a protein having FRNK activity and derivatives thereof as described above, including active fragments thereof. Conditions which will permit other DNAs which upon expression provide for FRNK or a protein having FRNK activity, to hybridize to DNA encoding FRNK as described herein, can be determined in accordance with known techniques. For example, hybridisation of such sequences may be carried out under conditions of reduced stringency, medium stringency or even stringent conditions (e.g., conditions represented by a wash stringency of 35-40% formamide with five times Denhardt's solution, 0.5% SDS and 1x SSPE at 37°C; conditions represented by a wash stringency of 40-45% formamide with five times Denhardt's solution, 0.5% SDS, and 1x SSPE at 42°C; and conditions represented by a wash stringency of 50% formamide with five times Denhardt's solution, 0.5% SDS and Ix SSPE at 42°C, respectively, to DNA encoding FRNK or a protein having FRNK activity disclosed herein in a standard hybridization assay. (See, e. g., J. Sambrook et al., Molecular Cloning, A Laboratory Manual (2nd Ed. 1989)). In general, sequences which code for FRNK or protein having FRNK activity will be at least 75% homologous, 85% homologous, or even 90 homologous or more to the sequence encoding FRNK disclosed herein and, preferably, encode a protein that retain the biological activity of FRNK as given above. Herein, FRNK, FRNK protein and a protein having FRNK activity are used in a synonymous manner if not explicitly indicated to the contrary. The same applies also to the use of the terms protein and polypeptide.

FRNK protein or FRNK encoding DNA can be of natural origin from any suitable species, including mammalian, more preferably human, or can be of synthetic origin.

The vector system which, particularly in combination with the aforementioned FRNK or a protein having FRNK activity and the nucleic acids coding therefor, allows for a very efficient gene therapeutic treatment of restenosis, restenosis related or/and stenosis related diseases, is the adeno-associated virus.

The adeno-associated virus is a member of the parvovirus group. The viruses comprise a circular single-stranded DNA molecule having a length of about 5600 bases. They may typically replicate and initiate a productive infectious cycle only when the cells are also infected by helper viruses such as adenoviruses, vaccinia or herpes virus. Apart from helper cells plasmid(s) providing the transacting factors can be provided and used to allow for the replication of adeno-associated viruses. A productive replication may also occur upon treatment of the cells with cytostatics such as Danuboblastin and/or radiation. Adeno-associated viruses comprise three different promoters, namely p5, p19 and p40, which are relevant for the regulation of gene expression.

The present invention is not limited to the use of a particular adeno-associated virus. However, particularly preferred is the use of adeno-associated virus type 2 (Rohr U et al, J. Virological Methods, 105 (2002): 265-275). Other adeno-associated viruses which can be used in the present invention are known to the one skilled in the art, e.g. AAV-1 to AAV-6 (Grimm D et al, Molecular Therapy, 2003 Vol 7 (6): 839-850)).

The present inventors have surprisingly found that the concerns expressed in the prior art, namely that adeno-associated virus vectors are only suitable for the transfection of just endothelial cells in the vaso vasorum (Lynch, CM, Circ. Res. 80: 497-505. 1997) are not justified. Rather to the contrary, intraluminal delivery using this kind of adeno-associated viruses comprising FRNK results in an unexpected highly efficient and persistent effect on inhibiting restenosis.

Preferably, the nucleic acid coding for FRNK and derivatives thereof is operably linked to at least one regulatory sequence directing the expression of FRNK, preferably the expression of FRNK in a cell susceptible to infection by said virus vectors. The regulatory sequence(s) directing the expression of FRNK is/are preferably promoters which may further comprise enhancers, further regulatory sequences such as operator sequences and the like. It is most preferable that the promoter is a promoter specific for smooth muscle cells. Such promoters can preferably be selected from the group comprising the "smooth muscle 22 alpha-actin promoter" (Solway J et al, J. Biol. Chem., 1995, 270: 13460-69). In addition, more universal promoters such as the CMV- or SV40- promoter (Rohr U et al, J Virol. Meth., 2002, 105: 265-275) can be employed.

It is within the present invention that both adeno-associated virus present as viral particles or as nucleic acid(s) coding for such adeno-associated virus can be used in any aspect of the present invention. Once prepared, the recombinant vector can preferably be reproduced by a) propagating the vector in a cell culture, the cell culture comprising cells that permit the growth and reproduction of the vector therein; and then b) collecting the recombinant vector from the cell culture, all in accordance with known techniques. The viral vectors collected from the culture may be separated from the culture medium in accordance with known techniques, and combined with a suitable pharmaceutical carrier for administration to a subject, or, used for the preparation of the coated stent and coated catheter, respectively, according to the present invention.

The adeno-associated virus coated implants as designed according to the present invention may be used for the prevention and/or treatment of a variety of diseases and is thus suitable to be subject to the manufacture of a respective medicament. The disease and the medicament for which the thus designed virus or virus encoding nucleic acid is used, are basically restenosis and stenosis, and any disease which is related to restenosis and stenosis, respectively. Also comprised are those diseases which arise from an undesired growth of smooth muscle cells, more preferably vascular smooth muscle cells. Preferably such diseases are coronary and/or peripheral vascular diseases. In principle, the various aspects of the present invention are applicable to both the arterial and the venous vascular system, although due to the molecular mechanism underlying the present invention the applicability to the arterial branch of the vascular system is even more advantageous.

With regard to the mode of action of FRNK and any protein having FRNK activity and its derivatives introduced into vascular smooth muscle cells, particularly the following diseases can be prevented and/or treated, whereby in principle such treatment can be done either prior or after the onset of the respective disease, or any treatment thereof such as ballooning has been performed: Acute coronary syndrome, peripheral arterial occlusion disease, vasculitis, thrombosis, carotis stenosis, stroke, any infarct, including but not limited to myocordial infarct, intestinal infarct, renal infarction, gangrene, arterial ulcera, venous thrombosis and amputation of any extremities.

In a preferred embodiment, any of these diseases is prevented and/or treated by the use of a stent or a catheter coated with the adeno-associated virus or a nucleic acid coding for such adeno-associated viruses as described herein. In other words, the particular diseases are preferably treatable if any part of the vascular system can be reached by a catheter or a stent.

In a further aspect the present invention is related to stents which are coated with an adeno-associated virus or a nucleic acid encoding for an adeno-associated virus as described herein, i.e. an adeno-associated virus which expresses and/or codes for FRNK, FRNK derivatives which retain the functional activity of FRNK and/or any protein having FRNK activity. Stents are as such known in the art and are typically endoprotheses which are used to bridge or maintain a lumen of a hollow viscus such as trachea, oesophagus and the vascular system. Typically, stents are self expanding upon having been placed in said hollow viscus. The placement is preferably done by endoscopic means as known in the art. Stents exist in many forms and the applicability of the present invention is not limited to distinct embodiments or forms of stents. Preferably, the adeno-associated virus or the nucleic acid coding therefor are adsorbed to the stent. Such adsorption may comprise covalent as well as non-covalent means. In case of covalent means, however, it is preferred that the covalent bonding of the adeno-associated virus to a surface of the stent is cleaved under physiological conditions, i. e. after placement of the stent at the respective location where the migration of the smooth muscle cells is to be inhibited. It is acknowledged that the immobilization of the adeno-associated virus to the stent can result in a characteristic release kinetics with the kinetics being controllable according to the condition to be treated. It is within the present invention that the respective adeno-associated virus may be released in the form of a burst to the cells so as to make sure that a particularly big number of smooth muscle cells are transfected. Given the fact that stents may remain in the body for longer periods of time, also a sustained release of the adeno-associated virus from the stent is, in principle, possible. This would allow that further generations of migrating smooth muscle cells would be infected by the thus later released adeno-associated viruses or the respective nucleic acid coding therefor.

The same considerations, possible use and design principles are also applicable to catheters. Accordingly, catheters may be coated by adeno-associated viruses or nucleic acid coding for such adeno-associated viruses as described herein. Compared to stents, however, the delivery of the adeno-associated viruses as described herein to the smooth muscle cells can be performed for a very limited period of time only, namely for the time the catheter is inserted into the hollow viscus, more particularly into the respective vessel(s), where the adeno-associated virus is to be delivered. The application of catheters is generally aiming at a rather short blocking of the lumen of the hollow viscus such as the lumen of a vessel, so that depending on the particular form of the catheter used, a rather short contact time between the smooth muscle cells and the adeno-associated viruses released from the catheter is realized. It is, however, acknowledged that there are also catheters available which do not block the flow of body fluid through the lumen of the hollow viscus into which the catheter is inserted. This would allow for a longer lasting contact between the catheter and the site of the vessel where the growth and more particularly the migration of the smooth muscle cells is to be inhibited by FRNK and its derivatives introduced via the adeno-associated virus. In principle, the same coating and adsorption and immobilization techniques as described for stents are applicable to the administration of the adeno-associated viruses as described herein via catheters.

A particular advantageous system to adsorb or immobilize the adeno-associated virus on a stent and catheter, respectively, is the use of a polymer. The adeno-associated virus is embedded in the respective polymer. Typically, the polymer exhibits a lattice structure which acts as a cage retaining the adeno-associated virus. The polymer is preferably one which can be degraded under physiological conditions, more particularly under conditions which prevail at the site where the adeno-associated virus is to be administered or delivered to. Alternatively, the polymer is nondegradable, remaining on the coated device, allowing the absorption of adeno-associated virus or various other formulations onto the surface. Preferably, the coating is such as to allow an immediate release of the adeno-associated virus to the smooth muscle cells. More preferably, the coating is at the surface of either the stent or the catheter which is different from the surface which is immediately contacted by the body fluid passing through the respective hollow viscus and more particularly the respective vessel. In a further aspect the present invention is related to the manufacture of a stent and/or catheter which is coated by an adeno-associated virus as described herein.

The invention will now be further illustrated by the figures and examples from which further aspects, embodiments, features and advantages of the present invention may be taken. More particularly
- Fig. 1: shows a schematic representation of the functional domains of FAK and FRNK;
- Fig. 2A: shows a diagram depicting the adeno-associated virus based vector with a multi-cloning site into which FRNK with a 3 x HA-tag was cloned;
- Fig. 2B: shows the amino acid sequence of FRNK as cloned into the adeno-associated virus depicted in Fig. 2A;
- Fig. 3A: shows a Western blot analysis of the expression of FAK and its phosphorylation in whole cell lysates of suspended and adherent human coronary smooth muscle cells upon stimulation by vitronectin;
- Fig. 3B: shows a diagram of the impact of vitronectin on haptotaxis of smooth muscle cells;
- Fig. 3C: shows a photograph of smooth muscle cells treated with bovine serum albumin and vitronectin, respectively, after staining with crystal violet;
- Fig. 4A: shows a photograph of smooth muscle cells which were infected with adeno-associated virus encoding for beta-galactosidase;
- Fig. 4B: shows a Western blot analysis of cell lysates of human smooth muscle cells after infection with a FRNK encoding adeno-associated virus; and
- Fig. 4C: shows a diagram depicting haptotaxis-migration of human smooth muscle cells upon stimulation with vitronectin and transfection with a FRNK expressing adeno-associated virus.

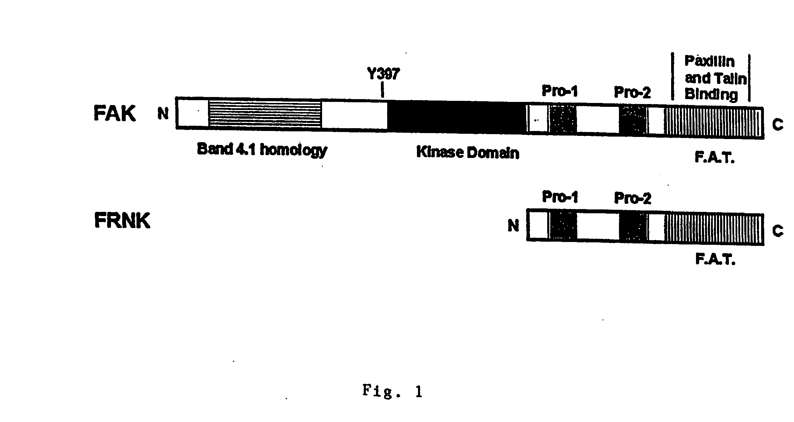

Fig. 1 shows a schematic representation of the functional domains of FAK and FRNK. FRNK comprises several domain, namely, the band 4.1 homology domain which is followed by the kinase domain and two proline-rich stretches (Pro-1 and Pro-2) that can serve as SH3-domain containing protein docking sites, such as p130Cas and Rho-GAP GRAF and the focal adhesion targeting domain, also referred to as FAT domain. Close to the kinase domain the tyrosine residue at position 397 is the target for the autophosphorylation of FAK which is a prerequisite for the signal transduction mediated by FAK as will be further outlined in example 2.

FRNK comprises the C-terminal stretch subsequent to the kinase domain of FAK. In the particular embodiment of FRNK as expressed in Fig. 1, FRNK comprises both the pro-1 and pro-2 stretch as well as the FAT domain.

The band 4.1 homology domain is involved in protein-protein interactions linking FAK to protein tyrosine kinases such as the EGF-receptor or others. The kinase domain as already described earlier is responsible for the signal transduction via phosphorylation, including autophosphorylation of FAK. The probably most important domain of the FAK is the focal adhesion targeting domain which mediates the localisation of FAK to integrins. The FAT domain comprises a bundle of 4-alpha helices which are associated through hydrophobic interactions. This stretch, particularly the FAT domain thereof, is expressed as a separate transcript in some cell types, including smooth muscle cells. This transcript is referred to as FAK-related non-kinase (FRNK). As FRNK comprises the FAT domain it is capable of competing with FAK for the respective binding sites. Given the lack of any kinase activity of FRNK compared to FAK, FRNK is a dominant-negative version of the endogenous FAK and is thus an efficient inhibitor to FAK dependent cellular events.

### Example 1: Construction of a FRNK expressing adeno-associated virus

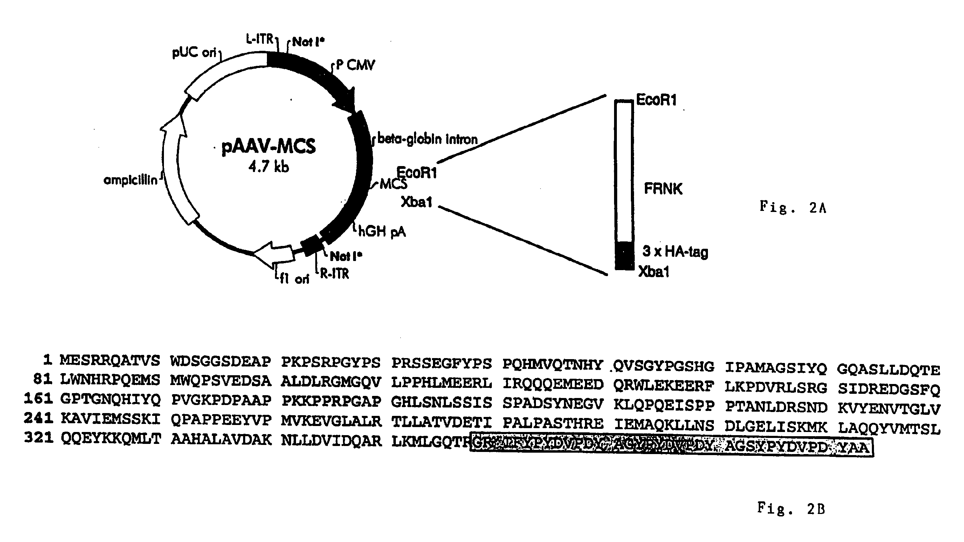

The coding sequence of murine FRNK, i. e. the amino acid sequence corresponding to amino acid 693 to 1050 of FAK as also depicted in Fig. 2B was amplified by PCR. The restriction sites for restriction enzymes EcoR1 and Xba1 were additionally introduced at the 5'end of the primers, respectively. The resulting PCR fragment was subsequently introduced into the vector pAAV-MCS which is part of the commercially available adeno-associated virus system of Stratagene, La Jolla, California. The insert did not only comprise the FRNK amino acid sequence but also a HA-tag at the 3'end which was introduced so as to allow for a specific detection of FRNK as expressed by the adeno-associated virus. The sequence of the vectors of the pAAV system are accessible through the following internet site: http://www.stratagene.com/vectors/selection/aav_vectors.html.

The vectors of the pAAV system were used for the production of infectious, replication deficient adeno-associated virus particles according to the supplied protocol employing human 293T cells using DMEM Medium containing 10% calf serum (PAA Laboratories, Linz, Austria) at 37°C, 5% CO₂. Typically, cells were kept in 10-cm-diameter tissue culture dishes containing 10 ml of this medium. Cell confluency was maintained between 20 - 90%. In parallel, the vector pAAV-LacZ of Stratagene was used containing the beta galactosidase (Lac Z)-gene of *Escherichia coli* in order to produce recombinant, LacZ-encoding AAV-particles. After repeated freeze-thawing of the transfected human 293T-cells and release of the FRNK-encoding virus particles, also referred to as AAV-FRNK, and the LacZ-encoding virus particles (AAV-LacZ), respectively, the titer of the infectious virus particles was determined. To that end, human vascular smooth muscle cells were infected with different dilutions of the virus preparation according to the protocol given below and 5 days later the number of lac Z-positive cells was determined. Therefrom the number of infectious particle per volume unit of the preparation could be determined. The primary human smooth muscle cells are, among others, commercially available from Clonetics.

The cloning strategy, more particularly the cloning vector is again depicted in Fig. 2A and the amino acid sequence cloned into the MCS in Fig. 2B.

### Example 2: Tyrosine phosphorylation of FAK in smooth muscle cells upon integrin simulation

In this experiment the importance of FAK as target in the inhibition of migration of smooth muscle cells and thus in restenosis was confirmed. FAK turned out to be one of the most strongly tyrosine phosphorylated proteins after simulation of smooth muscle cells with growth factors or proteins of the extracellular matrix such as vitronectin which were identified as activators for smooth muscle cell migration in connection with restenosis.

To characterize cellular proteins stimulated by attachment to the extracellular matrix, smooth muscle cells were serum-starved for 16 h in DMEM containing 0.5% calf serum, detached from the plate by limited trypsin digestion and kept in suspension at 37°C in DMEM containing 0.2% bovine serum albumin (suspension medium). After 1 h in suspension, cells were either pelleted and lysed in modified RIPA buffer (25 mM Hepes (pH 7.4), 0.1% SDS, 0.5% sodium deoxycholate, 1% Triton X-100, 150 mM NaCl, 20 mM MgCl₂, 10% glycerol, 10 mM sodium pyrophosphate, 100 mM NaF, 1 mM Na₃VO₄, and 10 µg/ml of each aprotinin, leupeptin, pefabloc and pepstatin) (Suspension sample) or plated onto a 10 cm cell culture dish coated for 16 h at 4°C with 5 ml of 1 µg/ml vitronectin in PBS, before the vitronectin-attached cells were lysed in modified RIPA buffer (VN-stimulated). Equivalent amounts of the cleared cell lysates were added to an equal volume of reducing 2x SDS sample buffer, the proteins were separated by SDS-PAGE, and transferred to PVDF membranes. Western blotting with anti-phosphotyrosine and anti-FAK antibodies, respectively, was performed as described (Schlaepfer, D. D. and Hunter T., Trends Cell Biol., 1998. 8 (4): p. 151-157).

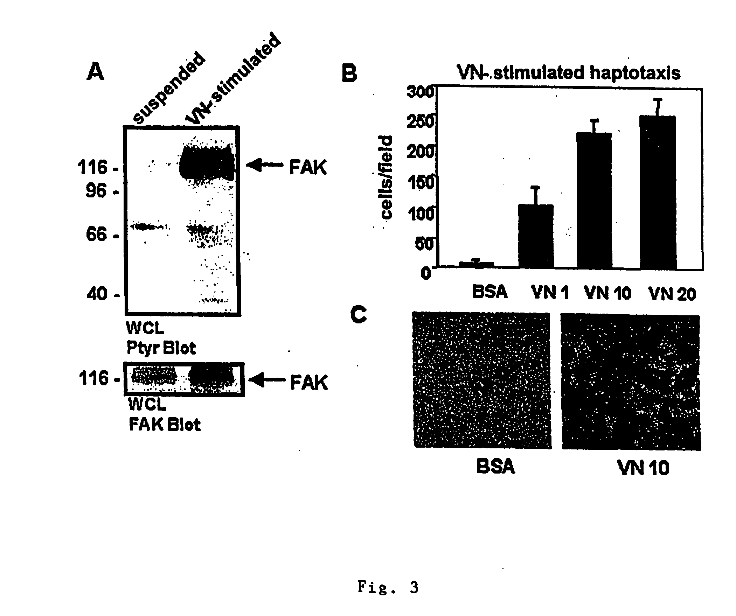

As depicted in Fig. 3A, the tyrosine phosphorylation of FAK is significantly increased upon stimulation of the cellular integrins by plating onto vitronectin (VN) compared to cells left in suspension without any integrin stimulus (SUS). In the top panel of Fig. 3A the phosphorylated FAK is detected in a Western blot analysis using an anti-phosphotyrosine antibody (Ptyr), i. e. an antibody which specifically detects phosphorylated tyrosine residues. The lower panel of Fig. 3A shows a Western blot analysis of the same samples using a monoclonal anti-FAK antibody (BD Biosciences, Heidelberg, Germany) confirming that the observed effects are not based on transcriptional or translational effects altering the level of FAK protein, but depend on the phosphorylation status of FAK. Both in the suspended as well as vitronectin-replated cells FAK was expressed at a similar level.

To analyze the migratory responses of primary human smooth muscle cells towards immobilized vitronectin, the cells were analysed in a modified Boyden chamber haptotaxis migration assay. To this end, Millicell chambers (8 µm pore size; Millipore, Bedford, MA) were coated on the lower side of the porous membrane with the indicated amounts of vitronectin for 2 h at room temperature and then placed in 24-well plates containing 400 µl of suspension medium. 1 x 10⁵ serum-starved smooth-muscle cells in 300 µl suspension medium were added into the chamber and allowed to migrate for 6 h at 37°C, 5% CO₂. Then cells remaining in the inner chamber were wiped off with a cotton-tip applicator and cells that had migrated to the vitronectin-coated surface on the lower side of the porous membrane of the Boyden chamber were fixed (37.5% ethanol, 25% acetic acid in H₂O) and stained with crystal violet. Stained cells in 4 random fields/chamber were counted under an inverse microscope at low power magnification (40x) and average cell numbers ± standard deviation are depicted in Figure 3B. The results show that vitronectin stimulates haptotaxis of smooth muscle cells in a concentration-dependent manner compared to chambers coated with bovine serum albumin. The importance of this study is based on the fact that the cells used for this purpose are primary human smooth muscle cells from coronary artery which are deemed to play a critical role in the phenomenon of restenosis.

The motogenic potential of vitronectin is further demonstrated by microphotographs of the lower membrane of two representative migration chambers from the haptotaxis migration experiment described above, where the smooth muscle cells at the lower membrane side of the Boyden chamber were fixed and stained using crystal violet after 6 hours of migration. Fig. 3C illustrates that cells exposed to BSA did not migrate and therefore no cells were visible on the lower side of the membrane, whereas the cells placed in Millicell chambers coated with vitronectin (here with a concentration of 10 µg/ml) showed a strong migratory response and therefore numerous smooth muscle cells stained with crystal violet were found on the lower side of the porous membrane (Figure 3C).

### Example 3: Inhibition of vitronectin-stimulated haptotaxis of smooth muscle cells upon infection with FRNK expressing adeno-associated virus

In this experiment, the adeno-associated virus based vectors produced in example 1 (AAV-LacZ and AAV-FRNK) were used. In order to prove the suitability of the virus for transfection, porcine smooth muscle cells as well as human smooth muscle cells were infected with AAV-LacZ. Therefore, smooth muscle cells were cultivated in 10 cm cell culture dishes containing 10 ml growth medium (SmBM (catalogue number CC-3181; CellSystems, St. Katharinen, Germany) with growth factor supplement SmGM-2 (catalogue number CC-4149; CellSystems, St. Katharinen, Germany) at 37°C and 5% CO₂ for one to two days. After the cell population reached a confluency of 20%, the medium was withdrawn and replaced by 5 ml DMEM with 2% heat inactivated calf serum (PAA Laboratories, Linz, Austria) and the cells were incubated with a multiplicity of infection (MOI) of 50 infectious virus particles per cell at 37°C and 5% CO₂ for 2 hours. Subsequently 5 ml growth medium were added to the culture. Five days later, the transduced smooth muscle cells were either fixed and stained for LacZ activity, lysed for determining protein expression or used in migration experiments.

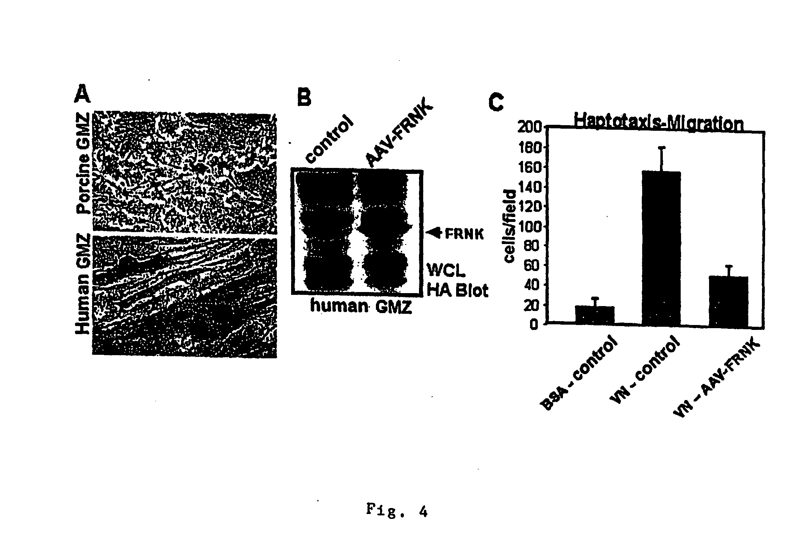

To analyse LacZ activity, the infected cells were fixed in 1.8% formalin, 0.05% glutaraldeyd in PBS for 5 min at room temperature two days after infection with the adeno-associated virus encoding beta-galactosidase (AAV-LacZ). Following fixation, the cells were incubated with LacZ staining solution (5 mM potassium ferricyanide, 5 mM potassium ferrocyanide, 1 mg/ml X-Gal, 2 mM MgCl₂ in PBS) for 2 h at 37°C, when LacZ-positive cells developed a blue staining. As can be seen in Fig. 4A, both porcine as well as human smooth muscle cells display blue staining as a result of beta-galactosidase activity demonstrating that they were successfully transduced using the adeno-associated viral vector.

To assess the ability of the FRNK-encoding adeno-associated virus to transduce primary smooth muscle cells, the cells were lysed in modified RIPA buffer five days after infection with AAV-FRNK and analysed by Western Blotting with anti-HA-tag antibodies (Santa Cruz Biotechnology, Santa Cruz, CA) as described above in Example 2.

Fig. 4B shows the expression of HA-FRNK in AAV-FRNK transduced smooth muscle cells (right lane), whereas uninfected control cells (left lane) do not show a corresponding protein band with the expected molecular weight of HA-FRNK.

To analyse the functional consequences of FRNK expression in human vascular smooth muscle cells a Boyden chamber haptotaxis migration assay with virus infected cells was carried out as described in example 2. As a migratory stimulus the chambers were coated on the lower side of the porous membrane either with bovine serum albumin (0.2% BSA in DMEM) as a negative control or with vitronectin (VN; 10 µg/ml in DMEM). Five days prior to the haptotaxis assay, cells were infected with the control adeno-associated virus (AAV-LacZ) or the adeno-associated virus encoding FRNK (AAV-FRNK) as described in example 2. As can be seen in Fig. 4C, AAV-LacZ-infected primary human smooth muscle cells respond to the immobilized vitronectin and migrate to the lower membrane surface. However, infection of the cells with AAV-FRNK and resulting expression of HA-FRNK in primary human smooth muscle cells efficiently decreases the vitronectin-stimulated haptotaxis migration close to the basal levels of migration seen in control cells in BSA-treated migration chambers.

### Example 4: Treatment of restenosis after PTCA and stent implantation by gene transfer of FRNK

### Animal model

The following is a protocol which is applied in order to prevent restenosis after PTCA and stent implantation, whereby restenosis is more particularly prevented through an adeno-associated virus-2 (AAV-2) mediated gene transfer of FRNK using a double balloon catheter. The animal model is pig.

The right Arteria carotis communis is prepared under full anaesthesia and mechanical ventilation. After introducing a 7F lock a selective angiography of the left coronary is performed by a 5F guiding catheter. A 3.0/10 mm stent is implanted into the proximal third of the Ramus interventricularis and the proximal third of the Ramus circumflexus of the left coronary. In order to induce a sufficient restenosis, a ratio of stent to artery of 1.3:1 is intended. After implantation of the stent a balloon perfusion catheter having two balloons is placed in the section of the blood vessel where the stent is located. The entire length of the stent plus two millimeters at each side is covered between the two occlusion balloons. To provide for an active perfusion of the coronary distal to the occluded section, arterial blood is used which is taken from the side port of lock 7F inserted in the Arteria carotis. The perfusion is performed by a roller pump. The blood is supplemented with a mixture of heparin and nitrates as a continuous infusion prior to the blood entering into the roller pump. In order to avoid damages to the vessel by the balloons, inflation is made at a minimum pressure (0.5 atm). 10 ml of a virus solution comprising adeno-associated virus coding 10⁸-10¹⁰ virus particles/ml for green fluorescence protein (AAV-GFP) and adeno-associated virus, respectively, coding for a fusion protein of FRNK and green fluorescence protein (AAV-FRNK-GFP) administered over ten minutes. Thereafter, the active perfusion catheter is removed, the Arteria carotis ligated and the cut closed.

For a period of four weeks after the surgery the animals receive a combination of Clopidogrel 75 mg and acetylsalicylic acid 100 mg per day per os. After four weeks, a further angiography is performed with subsequent removal of the organs. The heart is fixed with a formaldehyde solution in a Langendorff perfusion. After that the coronary sections comprising the stents are prepared and analysed in terms of histology and morphometry.

### Histology and morphometry

Subsequent to the afore-described trial with the stent implants having been in place for four weeks, morphometry of the restenosis, virus distribution in histological sections, formation of the neointima at the site of the balloons as well as toxicological analysis of organ damages and systemic virus release in the organs are assessed.

For morphometry, perfusion fixed sections of the coronary are embedded in methacrylate and sections prepared by means of a microtome. Planimetric morphometry is performed after HE staining for quantitative determination of restenosis.

In addition, the same sections are irradiated using fluorescent light (excitation wavelength 500 nm) in order to detect the green fluorescence protein (GFP) which was co-transfected by the virus and allows for determination of transfection efficacy. In addition, three sections of the vessels are analysed for FRNK as well as the co-transfected markers FLAG and HA using Western Blot analysis. The sample vessel segment is isolated immediately after removal of the organs. Blood and the stent are removed in ice cold buffer. The tissue is stored in liquid nitrogen prior to further processing for protein analysis. Similarly, tissue samples of adjunct segments of the blood vessel, the myocardium as well as of organs such as lung, liver, kidney and skeletal muscle are analysed for viral markers in order to determine local and systemic distribution. Blood samples are stored prior and subsequent to the trials and analysed for damages to the haematopoietic system, and for liver, heart, muscle and kidney parameters.

## Claims

1. Use of an adeno-associated virus, whereby the adeno-associated virus comprises
a) focal adhesion kinase-related non-kinase (FRNK) or a derivative thereof which retains the functional activity of FRNK,or
b) a nucleic acid encoding FRNK or a derivative thereof which retains the functional activity of FRNK, or
of a nucleic acid coding for an adeno-associated virus, whereby the nucleic acid comprises a nucleic acid encoding FRNK, for the coating of a stent and/or for the coating of a catheter.

2. The use according to claim 1, wherein FRNK comprises an amino acid sequence according to SEQ ID No. 1 or SEQ. ID. No. 2.

3. The use according to claim 1, wherein FRNK is encoded by a nucleic acid according to SEQ ID No. 3 or SEQ. ID. No. 4.

4. The use according to claim 1, wherein FRNK is encoded by a nucleic acid which hybridises to the nucleic acid sequence according to SEQ ID No. 3 or SEQ. ID. No. 4.

5. The use according to claim 1, wherein FRNK is encoded by a nucleic acid which, but for the degeneracy of the genetic code, would hybridise to the nucleic acid sequence according to SEQ ID No. 3 or SEQ. ID. No. 4.

6. Stent coated with an adeno-associated virus as described in any of the preceding claims or coated with a nucleic acid encoding the adeno-associated virus as described in any of the preceding claims.

7. Catheter coated with an adcno-associated virus as described in any of the preceding claims or a nucleic acid encoding the adeno-associated virus as described in any of the preceding claims.

8. Use of a stent according to claim 6 in the manufacture of a medicament or in the manufacture of a medical device.

9. Use of a catheter according to claim 7 in the manufacture of a medicament or in the manufacture of a medical device.

10. The use according to any of claims 8 and 9, whereby the medicament and/or the medical device is for the treatment and/or prevention of a coronary and/or peripheral vascular disease.

11. The use according to claim 10, wherein the coronary and/or peripheral vascular disease is selected from the group comprising restenosis, angiogenesis, angina pectoris, heart attack, acute coronary syndrome, peripheral arterial occlusion disease, vasculitis, thrombosis, carotis stenosis, stroke, infarction, gangrene, arterial ulcera, venous thrombosis and amputation of any extremities.

## Patentansprüche

1. Verwendung eines Adeno-assoziierten Virus, wobei der Adeno-assoziierte Virus umfasst:
a) focal adhesion kinase-related non-kinase (FRNK) oder ein Derivat davon, das die funktionelle Aktivität von FRNK beibehält, oder
b) eine Nukleinsäure, die für FRNK oder ein Derivat davon, das die funktionelle Aktivität von FRNK beibehält, codiert, oder
einer Nukleinsäure, die für ein Adeno-assoziiertes Virus codiert, wobei die Nukleinsäure, eine Nukleinsäure, die für FRNK codiert, umfasst, für das Beschichten eines Stents und/oder für das Beschichten eines Katheters.

2. Verwendung nach Anspruch 1, wobei FRNK eine Aminosäuresequenz gemäß SEQ.ID.No. 1 oder SEQ.ID.No. 2 umfassst.

3. Verwendung nach Anspruch 1, wobei FRNK von einer Nukleinsäure gemäß SEQ.ID.No. 3 oder SEQ.ID.No. 4 codiert wird.

4. Verwendung nach Anspruch 1, wobei FRNK von einer Nukleinsäure codiert wird, die mit der Nukleinsäuresequenz gemäß SEQ.ID.No. 3 oder SEQ.ID.No. 4 hybridisiert.

5. Verwendung nach Anspruch 1, wobei FRNK von einer Nukleinsäure codiert wird, die, ohne die Degeneriertheit des genetischen Codes, mit der Nukleinsäuresequenz gemäß SEQ.ID.No. 3 oder SEQ.ID.No. 4 hybridisieren würde.

6. Stent, der mit einem Adeno-assoziierten Virus, wie in einem der vorhergehenden Ansprüche beschrieben, oder mit einer Nukleinsäure, die für das Adeno-assoziierte Virus, wie in einem der vorhergehenden Ansprüche beschrieben, codiert, beschichtet ist.

7. Katheter, der mit einem Adeno-assoziierten Virus, wie in einem der vorhergehenden Ansprüche beschrieben, oder mit einer Nukleinsäure, die für das Adeno-assoziierte Virus, wie in einem der vorangehenden Ansprüche beschrieben, codiert, beschichtet ist.

8. Verwendung eines Stents nach Anspruch 6 für die Herstellung eines Medikaments oder für die Herstellung einer medizinischen Vorrichtung.

9. Verwendung eines Katheters nach Anspruch 7 für die Herstellung eines Medikaments oder für die Herstellung einer medizinischen Vorrichtung.

10. Verwendung nach Anspruch 8 oder 9, wobei das Medikament und/oder die medizinische Vorrichtung für die Behandlung und/oder Prävention einer koronaren oder periphären vaskulären Erkrankung ist.

11. Verwendung nach Anspruch 10, wobei die koronare und/oder periphäre vaskuläre Erkrankung ausgewählt ist aus der Gruppe umfassend Restenose, Angiogenese, Angina pectoris, Herzanfall, akutes Koronarsyndrom, periphäre Gefäßverschlusskrankheit, Vaskulitis, Thrombose, Karotisstenose, zerebrovaskulären Insult, Infarzierung, Gangrän, arterielle Ulcera, Venenthrombose und Amputationen jeglicher Gliedmaßen.

## Revendications

1. Utilisation d'un virus adéno-associé, dans laquelle le virus adéno-associé comprend :
a) une protéine non-kinase associée à la kinase de l'adhérence focale (FRNK) ou un dérivé de celle-ci qui conserve l'activité fonctionnelle de la FRNK, ou
b) un acide nucléique codant la FRNK ou un dérivé de celle-ci qui conserve l'activité fonctionnelle de la FRNK, ou
d'un acide nucléique codant pour un virus adéno-associé, utilisation dans laquelle l'acide nucléique comprend un acide nucléique codant la FRNK, pour le revêtement d'une endoprothèse vasculaire et/ou pour le revêtement d'un cathéter.

2. Utilisation selon la revendication 1, dans laquelle la FRNK comprend une séquence d'acides aminés selon la séquence SEQ ID No. 1 ou SEQ ID No. 2.

3. Utilisation selon la revendication 1, dans laquelle la FRNK est codée par un acide nucléique selon la séquence SEQ ID No. 3 ou SEQ ID No. 4.

4. Utilisation selon la revendication 1, dans laquelle la FRNK est codée par un acide nucléique qui s'hybride à la séquence d'acide nucléique selon la séquence SEQ ID No. 3 ou SEQ ID No. 4

5. Utilisation selon la revendication 1, dans laquelle la FRNK est codée par un acide nucléique qui, en prenant en compte la dégénérescence du code génétique, s'hybriderait à la séquence d'acide nucléique selon la séquence SEQ ID No. 3 ou SEQ ID No. 4.

6. Endoprothèse vasculaire revêtue d'un virus adéno-associé comme décrit dans l'une quelconque des revendications précédentes ou revêtue d'un acide nucléique codant le virus adéno-associé comme décrit dans l'une quelconque des revendications précédentes.

7. Cathéter revêtu d'un virus adéno-associé comme décrit dans l'une quelconque des revendications précédentes ou d'un acide nucléique codant le virus adéno-associé comme décrit dans l'une quelconque des revendications précédentes.

8. Utilisation d'une endoprothèse vasculaire selon la revendication 6 pour fabriquer un médicament ou pour fabriquer un dispositif médical.

9. Utilisation d'un cathéter selon la revendication 7 pour fabriquer un médicament ou pour fabriquer un dispositif médical.

10. Utilisation selon l'une quelconque des revendications 8 et 9, dans laquelle le médicament et/ou le dispositif médical est destiné au traitement et/ou à la prévention d'une maladie coronarienne et/ou vasculaire périphérique.

11. Utilisation selon la revendication 10, dans laquelle la maladie coronarienne et/ou vasculaire périphérique est choisie dans le groupe comprenant la resténose, l'angiogénèse, l'angine de poitrine, la crise cardiaque, le syndrome coronaire aigu, la maladie d'occlusion artérielle périphérique, le vascularite, la thrombose, la sténose carotidienne, une attaque, l'infarctus, la gangrène, l'ulcère artériel, la thrombose veineuse et l'amputation de toutes les extrémités.
